# EUROPÄISCHE PATENTANMELDUNG

(11) **EP 2 033 686 A2**
(43) Veröffentlichungstag der Anmeldung: **11.03.2009**
(21) Anmeldenummer: 08161615.3
(22) Anmeldetag: 01.08.2008
(51) Int. Cl.: A61Q 1/02, A61K 8/893

(54) **Foundation mit PEG/PPG-19/19 dimethicon**

(30) Priorität: 05.09.2007 DE 102007041978
(71) Anmelder: Beiersdorf AG, 20245 Hamburg (DE)
(72) Erfinder: Dippe, Rixa, 20255, Hamburg (DE); Fiedler, Dorothe, 22335, Hamburg (DE); Riedel, Heidi, 22083 Hamburg (DE); Klenner, Katja, 20255, Hamburg (DE)

(57) **Zusammenfassung**

Kosmetische Emulsion enthaltend
a) Farbstoffe und/oder Farb-Pigmente,
b) PEG/PPG-19/19 dimethicon,
c) Glycerin.

## Beschreibung

Die vorliegende Erfindung betrifft eine kosmetische Emulsion enthaltend Farbstoffe und/oder Farb-Pigmente sowie PEG/PPG-19/19 dimethicon und Glycerin.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.

Das Gesichts-Make-up soll der Gesichtshaut ein natürliches Aussehen verleihen, blasse Haut auffrischen und farbliche Unregelmäßigkeiten der Haut ausgleichen.

Neben Gesichtspudern und Rouge als pulverförmigen Kosmetika, werden hierzu cremeförmige Präparate, wie Tagescremes und Creme-Make-up auf Emulsionsbasis verwendet. Eine besondere Ausführungsform ist die so genannte Foundation. Als Foundation bezeichnet man dabei flüssige oder halbfeste Make-up-Präparate, die meist hautfarben sind und auf das Gesicht, insbesondere auf die Wangen aufgetragen werden. Sie verleihen diesen ein gleichmäßiges gesundes gebräuntes oder rötliches Aussehen.

Foundations können in unterschiedlicher Form aufbewahrt, dargereicht und aufgetragen werden. Neben einfachen Flaschen und Tuben, Überdruck- oder Pumpspendern, kommen in jüngerer Zeit auch Flaschen und Tuben mit aufgesetztem Applikator, insbesondere Pinseln und Schwämmen zum Einsatz. Derartige Systeme haben den großen Vorteil, dass sich die Zubereitungen direkt aus dem Vorratsbehältnis auf die Haut auftragen lassen, ohne dass die Anwenderin sich in irgend einer Form "die Hände schmutzig machen" muss.

Nachteilig am Stand der Technik ist jedoch der Umstand, dass die Zubereitungen häufig zu dünn- oder dickflüssig sind und damit von den Wangen fließen bzw. sich nur unter hohem Kraftaufwand (und damit schwer dosierbar) aus dem Vorratsbehältnis entnehmen lassen. Die Zubereitungen trocknen schnell auf dem Applikator ein, verkleben diesen und verstopfen die Entnahmeöffnung. Vor allem aber haben die Zubereitungen des Standes der Technik den großen Nachteil, sich mit den üblichen Applikatoren d.h. Pinsel und Schwamm nur ungleichmäßig auf die Haut auftragen zu lassen. Es entstehen bei dem Auftragen feine Streifen von ungleichmäßig dick aufgetragener Foundation auf der Haut. Derartige Streifen müssen dann von der Anwenderin wieder mit Hilfe der Finger gleichmäßig verteilt und damit aufgelöst werden. Kurzum: Die Hände werden dann doch wieder schmutzig. Die farbliche Abdeckung der Haut wird ungleichmäßig.

Es war daher die Aufgabe der vorliegenden Erfindung, die Nachteile des Standes der Technik zu beseitigen. Es war die Aufgabe, eine mit einem Applikator applizierbare Foundation zu entwickeln, die sich schon bei dem Auftrag mit dem Applikator gleichmäßig streifenfrei, gut dosierbar auftragen lässt. Dies sollte insbesondere auch dann noch möglich sein, wenn der Applikator schon vorher in unterschiedlichen Zeitabständen wiederholt zum Auftragen der Foundation benutzt wurde.

Nicht zuletzt war es die Aufgabe der vorliegenden Erfindung, ein einfach und kostengünstig herstellbares Foundation-Produkt zu entwickeln.

Überraschend gelöst werden die Aufgaben durch eine kosmetische Emulsion enthaltend
a) Farbstoffe und/oder Farb-Pigmente,
b) PEG/PPG-19/19 dimethicon,
c) Glycerin.

Es ist dabei erfindungsgemäß besonders bevorzugt, wenn es sich bei der erfindungsgemäßen Emulsion um eine Wasser-in-Silikonöl-Emulsion (W/S-Emulsion) handelt.

Zwar kennt der Fachmann die US 5066157, US 4747720, US 6488945, DE 3608955 und EP 1293440, doch konnten diese Schriften nicht den Weg zur vorliegenden Erfindung weisen. Ferner gibt es die noch unveröffentlichte DE 102006028549, die nicht zur Beurteilung der erfinderischen Tätigkeit heranzuziehen ist.

Erfindungsgemäß werden unter Wasser-in-Silikonöl-Emulsion Emulsionen mit einer inneren wässrigen Phase verstanden, deren Ölphase zu mindestens 50 Gew.-% aus Silikonölen besteht.

PEG/PPG-19/19 Dimethicone zeichnet sich durch die folgende Strukturformel aus 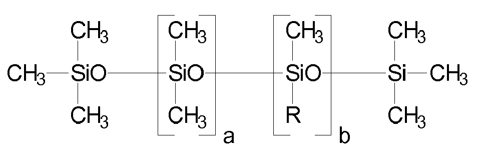 worin R = -CH₂CH₂CH₂O(EO)ₘ(PO)ₙR' und R' = -CH₃ oder -H darstellen.

PEG/PPG-19/19 Dimethicone kann vorteilhaft im Sinne der vorliegenden Erfindung auch in Form kommerziell erhältlicher öliger Vormischungen zur Anwendung kommen, beispielsweise in Mischungen mit Cyclomethicone. Eine solche vorteilhafte Vormischung ist beispielsweise unter der Handelsbezeichnung Dow Corning BY 11-030 bei der Fa. Dow Corning erhältlich.

Es ist erfindungsgemäß vorteilhaft, wenn die erfindungsgemäße Zubereitung PEG/PPG-19/19 dimethicon in einer Konzentration von 0,5 bis 20 Gewichts-%, bezogen auf das Gesamtgewicht der Zubereitung, enthält.

Es ist erfindungsgemäß bevorzugt, wenn die erfindungsgemäße Zubereitung PEG/PPG-19/19 dimethicon in einer Konzentration von 2 bis 5 Gewichts-%, bezogen auf das Gesamtgewicht der Zubereitung, enthält.

Es ist erfindungsgemäß vorteilhaft, wenn die erfindungsgemäße Zubereitung Farbstoffe und/oder Farbpigmente in einer Gesamtmenge von 1 bis 30 Gewichts-%, bezogen auf das Gesamtgewicht der Zubereitung, enthält.

Es ist erfindungsgemäß vorteilhaft, wenn die erfindungsgemäße Zubereitung Farbstoffe und/oder Farbpigmente in einer Gesamtmenge von 5 bis 20 Gewichts-%, bezogen auf das Gesamtgewicht der Zubereitung, enthält.

Die erfindungsgemäßen Pigmente können anorganisch oder organisch sein.

Bevorzugt im Sinne der vorliegenden Erfindung sind Pigmentmischungen aus Weiß-Pigmenten (z. B. Kaolin, Titandioxid oder Zinkoxid) und anorganischen Farbpigmenten (z. B. Eisenoxid-Pigmente, Chromoxide), wobei die Pigmente beschichtet ("gecoatet") oder unbeschichtet vorliegen können. Unter den Farbpigmenten sind Eisenoxide besonders bevorzugt.

Weißpigmente sind Pigmente, deren optische Wirkung vorwiegend auf nicht-selektiver Lichtstreuung beruht (siehe auch DIN 55944: 2003-11). Von den chemisch oft sehr ähnlichen Füllstoffen unterscheiden sich anorganische Weißpigmente vor allem durch ihre im allgemeinen höhere Brechzahl und - damit verbunden - ihr höheres Streuvermögen sowie nach DIN 55943: 2001-10 durch ihre Anwendung.

Erfindungsgemäß bevorzugte Weißpigmente zeigen keine Absorption im Bereich des sichtbaren Lichts, dafür aber ein hohes Streuvermögen, welches ein hohes Deckvermögen zur Folge hat. Das Streuvermögen ist umso größer, je größer die Differenz zwischen der Brechzahl des Weißpigmentes und der des umgebenden Mediums ist.

Erfindungsgemäß vorteilhafte Weißpigmente sind Titandioxide (Brechzahlen: 2,55 für Anatas und 2,75 für Rutil) und Zinkoxide (Brechzahl zwischen 1,95 und 2,1). Besonders bevorzugt ist Titandioxid.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäß vorteilhaft zusätzlich zu einem oder mehreren Weiß- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenförmiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenförmige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für erfindungsgemäß bevorzugte handelübliche Effektpigmente sind: Timiron® von Merck , Iriodin® von Merck (Perl- und Farbglanzpigmente für dekorative technische Anwendungen), Xirallic® von Merck (farbintensive Kristalleffektpigmente).

Ferner können die erfindungsgemäßen Zubereitungen vorteilhaft auch organische Farbpigmente enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weißpigmente sind ohne praktische Bedeutung.

Die Pigmente können vorteilhaft im Sinne der vorliegenden Erfindung auch in Form kommerziell erhältlicher öliger oder wässriger Vordispersionen zur Anwendung kommen.

Es ist ferner vorteilhaft im Sinne der vorliegenden Erfindung, wenn die erfindungsgemäße Zubereitung einen oder mehrere Farbstoffe enthält.

Die Farbstoffe können sowohl synthetischen als auch natürlichen Ursprungs sein.

Es ist erfindungsgemäß vorteilhaft, wenn die erfindungsgemäße Zubereitung Glycerin in einer Konzentration von 1 bis 20 Gewichts-%, bezogen auf das Gesamtgewicht der Zubereitung, enthält.

Es ist erfindungsgemäß bevorzugt, wenn die erfindungsgemäße Zubereitung Glycerin in einer Konzentration von 5 bis 10 Gewichts-%, bezogen auf das Gesamtgewicht der Zubereitung, enthält.

Erfindungsgemäß vorteilhafte Ausführungsformen der vorliegenden Erfindung sind dadurch gekennzeichnet, dass die erfindungsgemäße Zubereitung ein oder mehrere Füllstoffe enthält.

Erfindungsgemäß bevorzugt ist es in einem solchen Fall, wenn eine Kombination aus sphärischen und nichtsphärischen Füllstoffen eingesetzt wird.

Vorteilhaft im Sinne der vorliegenden Erfindung ist es, wenn die Zubereitung Füllstoffe in einer Gesamtmenge von 1 bis 10 Gewichts-%, bezogen auf das Gesamtgewicht er Zubereitung, enthält.

Bevorzugt im Sinne der vorliegenden Erfindung ist es, wenn die Zubereitung Füllstoffe in einer Gesamtmenge von 3 bis 7 Gewichts-%, bezogen auf das Gesamtgewicht er Zubereitung, enthält.

Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) sowie Lauroyllysine, Polymethylsilesquioxane, Polymethylmethacrylate, Polymethylmethacrylate Crosspolymer, Nylon, Talkum, beschichtetes Talkum, z.B. mit Dimethicone und Trimethylsiloxysilicate, Mica, Silica

Es ist erfindungsgemäß bevorzugt, wenn die erfindungsgemäße Zubereitung einen oder mehrere Füllstoffe gewählt aus der Gruppe der Verbindungen Nylon, Lauroyllysin (INCI: Lauroyl Lysine), Talkum, enthält.

Es ist erfindungsgemäß vorteilhaft, wenn die erfindungsgemäße Zubereitung als Silikonöle eine oder mehrere Verbindungen gewählt aus der Gruppe der Verbindungen der Dimethicone und Cyclomethicone enthält.

Die erfindungsgemäße Zubereitung kann darüber hinaus ein oder mehrere UV-Lichtschutzfilter und/oder kosmetische Wirkstoffe enthalten.

Erfindungsgemäß vorteilhafte Ausführungsformen der vorliegenden Erfindung sind dadurch gekennzeichnet, dass die Zubereitung einen oder mehrere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol) (Handelsname: Tinosorb M); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat ist doch = Ethylhexylsalicylat, oder; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer (INCI: Polysilicone-15); 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; den Merocyaninverbindungen; in einer Konzentration von 0,01 bis 40 Gewichts-% bezogen auf das Gesamtgewicht der Zubereitung. Dabei sind Konzentrationen von 1 bis 20 Gewichts-%, bezogen auf das Gesamtgewicht der Zubereitung erfindungsgemäß bevorzugt.

Erfindungsgemäß vorteilhaft enthält die erfindungsgemäße Zubereitung eine oder mehrere Verbindungen gewählt aus der Gruppe der Verbindungen Folsäure, D-Biotin, Coenzym Q10, alpha-Glucosylrutin, Carnitin, natürliche und/oder synthetische Isoflavonoide, Genistein, Flavonoide, Carotinoide, Kreatin, Kreatinin, Taurin, Ascorbinsäure und Derivate, Tocopherol und seine Ester.

Dabei ist der Einsatz von Tocopherolacetat erfindungsgemäß bevorzugt.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Elektrolyte wie besonders bevorzugt Salze wie Natriumchlorid und /oder Magnesiumsulfat.

Erfindungsgemäß bevorzugt enthält die erfindungsgemäße Zubereitung organische Carbonate, insbesondere Propylencarbonat und/oder Butylencarbonat, wobei der Einsatz von Propylencarbonat erfindungsgemäß besonders bevorzugt ist.

Derartige Carbonate werden erfindungsgemäß vorteilhaft in einer Konzentration von 0,02 bis 0,20 Gewichts-%, bezogen auf das Gesamtgewicht der Zubereitung eingesetzt.

Vorteilhafte Ausführungsformen der vorliegenden Erfindung sind dadurch gekennzeichnet, dass die Zubereitung Diazolidinylurea, Phenoxyethanol und/oder Parabene (Methylparaben, Ethylparaben, Propylparaben und Butylparaben, Isobutylparaben) in einer Gesamtkonzentration von größer oder gleich 0,25 Gewichts-%, bezogen auf das Gesamtgewicht der Zubereitung, enthält.

Erfindungsgemäß vorteilhaft enthält die erfindungsgemäße Zubereitung ein oder mehrere Parfümstoffe.

Es ist ferner vorteilhaft im Sinne der vorliegenden Erfindung, wenn die Zubereitung einen oder mehrere Filmbildner bzw. Polymere enthält, insbesondere um einen nachvollziehbaren Straffungseffekt auf der Haut zu erzielen, der durch deren Filmbildungseigenschaften hervorgerufen werden kann. Gleichzeitig dienen die Filmbildner zur Fixierung der Pigmente auf der Haut, um einen entsprechende lang anhaltenden Effekt und eine Transfer-Resistenz zu erzielen Hierzu eignen sich z. B. Trimethylsiloxysiliacte beispielsweise auch in Abmischung mit Dimethicone, Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF), C₂₀₋₄₀ Carbonsäure mit Polyethylen (Performacid 350 von der Fa. New Phase Technologies) sowie Filmbildner aus der Gruppe der Polymere auf Basis von Polyvinylpyrrolidon (PVP):

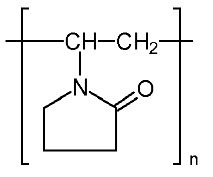

Besonders bevorzugt werden Copolymere des Polyvinylpyrrolidons eingesetzt, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Insbesondere bevorzugt sind wasserlösliche Polymere wie Vinylpyrrolidon/Vinylacetat- (VP/VA-) Copolymere und Natriumpolystyrensulfonat.

Es ist vorteilhaft im Sinne der vorliegenden Erfindung die Konzentrationen an Filmbildnern (eine oder mehrere Verbindungen) aus dem Bereich von 0,1 bis 5 Gew.-%, besonders bevorzugt wie 0,1 bis 3 Gew.-% - jeweils bezogen auf das Gesamtgewicht der Zubereitungen - zu wählen

Erfindungsgemäß sind die folgenden Rezepturen vom Schutzbereich der vorliegenden Erfindung ausgenommen:

| | **1** | **2** | **3** | **4** | **5** | **6** |
|---|---|---|---|---|---|---|
| | **Gew.-%** | **Gew.-%** | **Gew.-%** | **Gew.-%** | **Gew.-%** | **Gew.-%** |
| Wasser | ad 100 | ad 100 | ad 100 | ad 100 | ad 100 | ad 100 |
| Zitronensäure | | 0.1 | | | | |
| Alcohol Denat. | | | 10 | | 2,5 | 5 |
| Cetyl PEG/PPG-10/1 Dimethicon | | 4 | 1,5 | 2 | | |
| PEG/PPG-19/19 Dimethicone | 2 | 0,5 | 1,5 | 1 | 1,5 | 3 |
| PEG-30 Dipolyhydroxystearate | | | 0,5 | | | |
| Magnesium Sulfat | | | 1 | | | |
| Disteardimonium Hectorit | 0,5 | 0,5 | 0,25 | 0,25 | 0,25 | 0,5 |
| Natrium Chlorid | 2 | 2 | | 2 | 2 | 2 |
| Propylen Carbonat | 0,12 | 0,12 | 0,05 | 0,06 | 0,06 | 0,12 |
| Caprylic/Capric Triglycerid | | | | | | 4 |
| Dicaprylyl Ether | | | | | | 4 |
| Dicaprylyl Carbonate | 3 | | | 5 | 8 | |
| Dimethicon | | 3 | | | | 2 |
| Cyclomethicon | 15 | 17 | 30 | 19 | 13 | 15 |
| Dimethicon + Trimethylsiloxysilicat | | | | 3,5 | 3,5 | |
| Dimethicon + Dimethicon Crosspolymer | 6 | 4 | 6 | | | |
| Cyclomethicon + Dimethiconol | | 2 | | | | |
| Avocadoöl | | | 2 | | | |
| Aprikosenkernöl | | | 1 | | | |
| Squalan | | 4 | | 2 | 0,5 | |
| Lecithin | | | 1 | | | |
| Shea Butter | 1 | | | | | 1 |
| Myristyl Lactat | 1 | | | | | |
| Silica | | | 1 | | | |
| Aluminum Starch Octenylsuccinat | 3 | | 2 | | | 1 |
| Lauroyl Lysin | 1 | 3 | 1 | 2 | 2 | 4 |
| Talc | | 1 | | | | |
| Methyl Methacrylat Crosspolymer | | 1 | | | | |
| Nylon | | 2 | | 4 | 8 | |
| Mit Dimethylimidazolidinon modifizierte Reisstärke | | | 2 | | | |
| [Talkum beschichtet mit Dimethicon + Trimethylsiloxysilicat] | | | | 1 | | |
| Polymethylsilsesquioxan | | | 3 | | | 2 |
| VP/VA Copolymer | | 0,1 | | | | 0,2 |
| VP/Hexadecen Copolymer | | | | 0,5 | | 1,5 |
| Sodium Polystyren Sulfonat | 0,5 | 1 | 1 | | 0,4 | 0,5 |
| Titandioxid (Cl 77891) | 9 | 6 | 7 | 6 | 7 | 5 |
| Farbpigmente (Cl77492, 77491,77499,77007) | 4,5 | 5 | 6 | 7,5 | 5,5 | 6 |
| Beschichtetes Silica 2 | 3 | 2 | | 1 | 0,5 | |
| Effektpigmente (z.B. beschichtetes Mica) 3 | | 5 | 0,5 | | | 3,5 |
| Glycerin | 10 | 5 | 7 | 12,5 | 5 | 5 |
| Natrium Hyaluronat | | 1 | | | | 2 |
| Ethylhexyl Methoxycinnamat + BHT | 1 | 5 | | | | 1 |
| Titanium Dioxid | 3 | | 1 | | | 1 |
| Wasser + Glycerin + Lecithin + Butylene Glycol + Sodium Styrene/Acrylates Copolymer | 3 | 5 | | | | 0,5 |
| Ginkgo Biloba | | | 2 | | | |
| Vitamin E Acetat | 1,5 | 1 | 1,5 | 0,5 | 0,5 | 1,5 |
| Vitamin A Palmitat | 0,1 | | 0,5 | | | |
| Ubiquinon, Q10 | | 0,025 | | | 0,2 | |
| Biosaccharide Gum-1 | | | | | | 1 |
| Vitamin C Phosphate | | 0,25 | | 0,1 | | 0,1 |
| Calcium Pantothenat | | 0,5 | | | | |
| Glycin | 0,1 | | | | 0,7 | |
| Phenoxyethanol + Paraben | 1 | | 0,5 | | | 0,7 |
| Tetrasodium Iminodisuccinat | 0,2 | | | | | |
| Diazolidinyl Urea | 0,2 | 0,3 | | 0,3 | 0,3 | |

| | | | | | | |
|---|---|---|---|---|---|---|
| ^{2 z.B.} Ronasphere LDP von Merck (Silica + Titanium Dioxide + Iron Oxides) ^{3 z.B..} Timiron von Merck (Mica & Cl 77891) | | | | | | |

Die erfindungsgemäße Zubereitung wird erfindungsgemäß vorteilhaft in einem Behälter aus Kunststoffen der Polyolefingruppe, z.B. Polyethylen (PE) oder Polypropylen (PP) aufbewahrt und dargereicht, da diese eine gute Quetschbarkeit zur Entnahme der Zubereitung gewährleisten.

Es ist erfindungsgemäß bevorzugt, wenn die Behälterwand als Mehrlagenmaterial gefertigt ist. Erfindungsgemäß besonders bevorzugt ist ein Mehrlagenmaterial aus Polyethylen verbunden mit dem Barriere-Kunststoff EVOH.

Erfindungsgemäß bevorzugt stellt der erfindungsgemäße Behälter eine Tube dar.

Auf der Entnahmeöffnung des erfindungsgemäßen Behälters ist erfindungsgemäß bevorzugt ein Applikator aufgesetzt. Der Applikator ist dabei entweder abnehmbar oder nichtabnehmbar mit dem Behälter verbunden.

Es ist erfindungsgemäß bevorzugt, wenn als Applikator ein Pinsel oder Schwamm verwendet wird.

Der Pinsel-Applikator orientiert sich an den Werkzeugen der professionellen Kosmetiker, die ja auf beste Ergebnisse Wert legen. Die Pinselhaare sind erfindungsgemäß vorteilhaft aus Takelon®.

Es ist erfindungsgemäß vorteilhaft, wenn die Pinselhaare nicht gerade geschnitten sind, sondern im Profil eine Verjüngung jeder einzelnen Faser aufweisen.

Es ist vorteilhaft im Sinne der vorliegenden Erfindung, wenn die Pinselbündelung selbst in flacher ovaler Form ausgelegt ist und sich auch im Ganzen zur Spitze hin verjüngt. Dies führt zu einem besonders weichen und angenehmen Auftragegefühl, als auch zu einem besonders gleichmäßigem Auftrag der Zubereitung. Erfindungsgemäß vorteilhaft sind Pinselhaarlängen (ab Fassung) von 15-20mm.

Der Schwamm-Applikator besteht erfindungsgemäß vorteilhaft aus Nitrilbutadien-Rubber mit einer optimalen Oberfläche für den Auftrag von flüssigen Foundations. Er kann erfindungsgemäß vorteilhaft zweilagig hergestellt sein, mit einer unteren Schicht die eine höhere Dichte hat, also für die Zubereitung weniger durchlässig ist, und einer oberen Schicht, die eine geringere Dichte aufweist und das Produkt gut aufnimmt sowie eine gute Verteilbarkeit auf der Haut erlaubt. Dadurch erreicht man überraschenderweise eine bessere Dosierbarkeit und geringeren Produktverbleib im Schwamm selbst. Eine abgerundet-rechteckige Form des Schwamms erlaubt eine gute Erreichbarkeit aller Partien im Gesicht.

Es ist erfindungsgemäß vorteilhaft, den Produktaustritt aus dem Behälter nur zum Zeitpunkts des Gebrauchs freizugeben. Dies kann vorteilhaft durch eine mechanische Schließvorrichtung bewerkstelligt werden, die durch Drehen des Applikator-Aufsatzes um, z.B. eine viertel oder halbe Drehung den Behälter zum Applikator hin öffnet und schließt.

Erfindungsgemäß vorteilhaft wird der Applikator nach außen hin mit einem Schraub- oder Schnapp-Deckel verschlossen.

Erfindungsgemäß vorteilhaft ist ein Behälter, enthaltend eine erfindungsgemäße Zubereitung, dessen Öffnung mit einem Aufsatz versehen, der erlaubt, dass die Zubereitung mittels Schwamm- oder Pinsel direkt aufgetragen werden kann.

Erfindungsgemäß bevorzugt ist eine Tube aus Kunststoffen der Polyolefingruppe mit einem Pinselapplikator dessen Pinselhaare bevorzugt im Profil eine Verjüngung jeder einzelnen Faser aufweisen, enthaltend eine erfindungsgemäße Zubereitung.

Erfindungsgemäß bevorzugt ist eine Tube aus einem Mehrlagenmaterial aus Polyethylen verbunden mit dem Barriere-Kunststoff EVOH mit einem Pinselapplikator dessen Pinselhaare bevorzugt im Profil eine Verjüngung jeder einzelnen Faser aufweisen, enthaltend eine erfindungsgemäße Zubereitung.

Erfindungsgemäß bevorzugt ist eine Tube aus Kunststoffen der Polyolefingruppe mit einem Schwamm-Applikator aus Nitrilbutadien-Rubber der, bevorzugt, zweilagig aufgebaut ist mit einer unteren Schicht die eine höhere Dichte hat, und einer oberen Schicht, die eine geringere Dichte aufweist, enthaltend eine erfindungsgemäße Zubereitung.

Erfindungsgemäß bevorzugt ist eine Tube aus einem Mehrlagenmaterial aus Polyethylen verbunden mit dem Barriere-Kunststoff EVOH mit einem Schwamm-Applikator aus Nitrilbutadien-Rubber der, bevorzugt, zweilagig aufgebaut ist mit einer unteren Schicht die eine höhere Dichte hat, und einer oberen Schicht, die eine geringere Dichte aufweist, enthaltend eine erfindungsgemäße Zubereitung.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **1** | **2** | **3** | **4** |
|---|---|---|---|---|
| | Gew.-% | Gew.-% | Gew.-% | Gew.-% |
| Wasser | Ad 100 | Ad 100 | Ad 100 | Ad 100 |
| Glycerin | 7 | 5 | 5 | 10 |
| Cyclomethicon + PEG/PPG-19/19 Dimethicon | 4 | 4 | 2 | 5 |
| Propylene Carbonat | 0,05 | 0,06 | 0,06 | 0,03 |
| Disteardimonium Hectorite | 0,25 | 0,4 | 0,2 | 0,1 |
| Sodium Chloride | 2 | | 1 | 2 |
| Squalan | 0,5 | | 0,5 | |
| Dicaprylyl Carbonate | | 5 | | 5 |
| Cyclomethicone | 10 | 12 | 20 | 15 |
| Dicaprylyl Ether | 2 | | | |
| Caprylic/Capric Triglycerid | 2 | | | |
| Dimethicon | 2 | 3 | | 4 |
| Myristyl Laktat | | | 1 | |
| Lecithin | 1 | | 0,5 | |
| Dimethicon + Trimethylsiloxysilikat | | | 3,5 | 2 |
| Lauroyl Lysine | 5 | | 2,5 | 3 |
| Talkum | 2 | 3 | 0,5 | |
| Nylon 6/12 | | 3 | 2 | 4 |
| Silica | | 3 | 4 | 3 |
| Dimethicon + Dimethicon Crosspolymer | 6 | 4 | | |
| Methyl Methacrylat Crosspolymer | | | 1 | 2 |
| Polymethylsilsesquioxan | 2 | 1 | | |
| VP/VA Copolymer | 0,1 | | | 0,2 |
| VP/Hexadecen Copolymer | 0,1 | | 0,5 | |
| Titandioxid (Cl 77891) | 6 | 3 | 3 | 9 |
| Farbpigmente (Cl 77492 + Cl 77491 + Cl 77499) | 5 | 5 | 2 | 7 |
| Effektpigmente (z.B. beschichtetes Mica) | | 3 | 1 | |
| Octocrylen | 2 | 5 | | |
| Titandioxid | 1 | 2 | | |
| Sodium Ascorbyl Phosphate | | | 0,1 | 0,2 |
| Tocopheryl Acetate | 0,5 | 0,5 | 1 | 1,5 |
| Ubiquinon, Q10 | | 0,05 | | |
| Phenoxyethanol + Parabene | 1 | 0,7 | | |
| Tetrasodium Iminodisuccinat | | 0,2 | | |
| Diazolidinyl Urea | | | 0,3 | 0,3 |
| Parfum | 0,5 | 0,5 | 0,5 | 0,5 |
| Behältermaterial | PE/Haftvermittler/EVOH/Haftvermittler/PE | | Polyethylen | Polypropylen |
| Applikator | Pinsel, desse Fasem aus Takelon® bestehen und 17mm sichtbare Länge haben. | | Schwamm aus Nitrilbutadie n-Rubber (NBR) mit einer Dichte von <0,2g/cm³ | Schwamm aus Nitrilbutadien-Rubber (NBR), wobei die untere Lage eine höhere Dichte aufweist als die obere Lage. |

## Patentansprüche

1. Kosmetische Emulsion enthaltend
a) Farbstoffe und/oder Farb-Pigmente,
b) PEG/PPG-19/19 dimethicon,
c) Glycerin.

2. Kosmetische Emulsion nach Anspruch 1, **dadurch gekennzeichnet, dass** es sich um eine Wasser-in-Silikonöl-Emulsion handelt.

3. Kosmetische Zubereitung nach einem der vorhergehenden Ansprüche, **dadurch gekennzeichnet, dass** die Zubereitung PEG/PPG-19/19 dimethicon in einer Konzentration von 0,5 bis 20 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung Farbstoffe und/oder Farbpigmente in einer Gesamtmenge von 1 bis 30 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung Farbstoffe und/oder Farbpigmente gewählt aus der Gruppe der folgenden Verbindungen enthält: Weiß-Pigmenten (Kaolin, Titandioxid oder Zinkoxid) und anorganischen Farbpigmenten (Eisenoxid-Pigmente, Chromoxide), wobei die Pigmente beschichtet ("gecoatet") oder unbeschichtet vorliegen können.

6. Kosmetische Emulsion nach einem der vorhergehenden Ansprüche, **dadurch gekennzeichnet, dass** die Zubereitung ein oder mehrere Füllstoffe enthält.

7. Kosmetische Emulsion nach einem der vorhergehenden Ansprüche, **dadurch gekennzeichnet, dass** die Zubereitung Füllstoffe in einer Gesamtmenge von 1 bis 10 Gewichts-%, bezogen auf das Gesamtgewicht er Zubereitung, enthält.

8. Kosmetische Emulsion nach einem der vorhergehenden Ansprüche, **dadurch gekennzeichnet, dass** die Zubereitung einen oder mehrere Füllstoffe gewählt aus der Gruppe der Verbindungen Nylon, Lauroyllysin, Talkum enthält.

9. Kosmetische Zubereitung nach einem der vorhergehenden Ansprüche, **dadurch gekennzeichnet, dass** die Zubereitung als Silikonöle eine oder mehrere Verbindungen gewählt aus der Gruppe der Verbindungen der Dimethicone und Cyclomethicone enthält.

10. Behälter, enthaltend eine Zubereitung nach einem oder mehreren der vorhergehenden Ansprüche, dessen Öffnung mit einem Aufsatz versehen, der erlaubt, dass die Zubereitung mittels Schwamm- oder Pinsel direkt aufgetragen werden kann.
